# EUROPEAN PATENT APPLICATION

(11) **EP 4 074 338 A1**
(43) Date of publication of application: **19.10.2022**
(21) Application number: 20899757.7
(22) Date of filing: 14.12.2020
(51) Int. Cl.: A61K 39/395, A61K 47/26, A61K 47/18, A61K 47/22, A61K 9/00, A61P 35/00, C07K 16/28, A61K 39/00

(54) **STABLE ANTI-PD-1 ANTIBODY PHARMACEUTICAL PREPARATION**

(30) Priority: 13.12.2019 KR 20190167147
(71) Applicant: Samsung Bioepis Co., Ltd., Yeonsu-gu Incheon 21987 (KR)
(72) Inventor: JUNG, Young Seok, Incheon 22008 (KR); HONG, Ja Hye, Incheon 21982 (KR); JOO, Kyung Hee, Incheon 21982 (KR); HA, Young Wook, Incheon 21982 (KR); OH, In Young, Incheon 21982 (KR); KIM, In Ae, Seoul 05090 (KR)
(74) Representative: Boult Wade Tennant LLP
(86) International application number: PCT/KR2020/018247
(87) International publication number: WO 2021/118321

(57) **Abstract**

The present disclosure relates to a stable anti-PD-1 antibody pharmaceutical formulation and a method for preparing the same, the pharmaceutical formulation comprising: an anti-PD-1 antibody or an antigen binding fragment thereof; and a stabilizer, and not comprising a buffer.

## Description

### TECHNICAL FIELD

The present disclosure relates to stable anti-PD-1 antibody pharmaceutical formulations and preparation methods thereof.

### BACKGROUND

To enhance pharmaceutical stability, demands for development of stable anti-PD-1 antibody pharmaceutical formulations are rising. To attain pharmaceutical stability, an anti-PD-1 antibody pharmaceutical formulation includes a buffer, a stabilizer, and a surfactant.

However, some buffers are prone to self-decomposition under stress conditions, adversely affecting the stability of antibody medicament. In a case of a phosphoric acid buffer, a pH level may not be maintained in a freezing environment, and thus the stability of antibody may be affected.

Accordingly, there remains a demand for development of formulations capable of stabilizing an antibody protein even without including a buffer, which may potentially affect the stability of an anti-PD-1 antibody pharmaceutical formulation.

### DESCRIPTION OF EMBODIMENTS

### TECHNICAL PROBLEM

An aspect provides a stable anti-PD-1 antibody pharmaceutical formulation comprising: (a) an anti-PD-1 antibody or an antigen binding fragment thereof; and (b) a stabilizer, wherein the formulation does not comprise a buffer and has a pH of about 4.5 to about 6.5.

Another aspect provides a method for treating a cancer, comprising administering the pharmaceutical formulation to a subject.

Still another aspect provides a method for preparing the pharmaceutical formulation.

Additional aspects will be set forth in part in the description which follows and, in part, will be apparent from the description, or may be learned by practice of the presented embodiments of the disclosure.

### SOLUTION TO PROBLEM

Unless otherwise stated, all technical terms used in this specification have the meanings that are commonly understood by those skilled in the art. In addition, desired methods or samples are set forth herein, but methods or samples similar or equivalent thereto may also be included in the scope of the present disclosure. In addition, numeral values used in this specification are considered to include the meaning of "about" even though not explicitly stated. The contents of all publications listed in this specification are incorporated herein by reference in their entireties.

An aspect provides a stable anti-PD-1 antibody pharmaceutical formulation comprising: (a) an anti-PD-1 antibody or an antigen binding fragment thereof; and (b) a stabilizer, wherein the formulation does not comprise a buffer and has a pH of about 4.5 to about 6.5.

Unlike generally known pharmaceutical formulations comprising an antibody, the pharmaceutical formulation does not comprise a buffer, but may be maintained at a pH of 4.5 to 6.5, which is a desired environment for preservation of an anti-PD-1 antibody, specifically at pH of about 5.0 to about 5.5, which is a more desired environment for preservation of an anti-PD-1 antibody. The pH of the pharmaceutical formulation may be, for example, pH 4.5 to 6.3, pH 4.8 to 6.3, pH 5 to 6.3, pH 5.2 to 6.3, pH 4.5 to 6.0, pH 4.8 to 6.0, pH 5.0 to 6.0, pH 5.2 to 6.0, pH 4.5 to 5.8, pH 4.8 to 5.8, pH 5.0 to 5.8, pH 5.2 to 5.8, pH 4.5 to 5.6, pH 4.8 to 5.6, pH 5.0 to 5.6, pH 5.2 to 5.6, pH 4.9 to 5.5, pH 4.9, pH 5.0, pH 5.1, pH 5.2, pH 5.3, pH 5.4, or pH 5.5. In a specific embodiment, the pharmaceutical formulation may be at a pH of about 4.5 to about 5.5. In another specific embodiment, the pH of the pharmaceutical formulation may be about 5.0 to about 5.5.

The pH of the pharmaceutical formulation may be adjusted by a general method known in the art. For example, the pH of the pharmaceutical formulation may be adjusted by addition of an acid (e.g., HCI) or a base (e.g., NaOH), but not limited thereto.

The term "antibody" refers to an any type of antibody having the activity to specifically bind to PD-1. The antibody includes a monoclonal antibody, a polyclonal antibody, a humanized antibody, a human antibody and a chimeric antibody. The antibody may be a pembrolizumab.

The term "antigen binding fragment" is a fragment that is capable of binding to a PD-1 antigen in an anti-PD-1 antibody, including, for example, Fab fragment, F(ab')₂ fragment, Fc fragment, or scFv fragment, but not limited thereto. The term "pembrolizumab antibody" is a humanized antibody used in cancer immunotherapy and is currently marketed under the trade name KEYTRUDA^{®} as one of various commercialized products. The pembrolizumab may be used in treating melanoma, lung cancer including non-small cell lung cancer (NSCLC), head and neck cancer including head and neck squamous cell cancer (HNSCC), Hodgkin lymphoma including classical Hodgkin lymphoma (cHL), urothelial cancer, renal cell carcinoma, stomach cancer, microsatellite instability-high cancer (MSI-H), mismatch repair deficient (dMMR) solid cancer, cervical cancer, liver cancer, Merkel cell carcinoma (MCC), or the like. The pembrolizumab may also include biosimilars or biobetters of active pembrolizumab present in commercially available KEYTRUDA products. The pembrolizumab may include a heavy chain variable region having at least one amino acid sequence selected from the group consisting of SEQ ID NOs: 1 to 3 and a light chain variable region having at least one amino acid sequence selected from the group consisting of SEQ ID NOs: 4 to 6. The pembrolizumab may include a heavy chain CDR1 having the amino acid sequence of SEQ ID NO: 1 (NYYMY), a heavy chain CDR2 having the amino acid sequence of SEQ ID NO: 2 (GINPSNGGTNFNEKFK), a heavy chain CDR3 having the amino acid sequence of SEQ ID NO: 3 (RDYRFDMGFDY), a light chain CDR1 having the amino acid sequence of SEQ ID NO: 4 (RASKGVSTSGYSYLH), a light chain CDR2 having the amino acid sequence of SEQ ID NO: 5 (LASYLES), and a light chain CDR3 having the amino acid sequence of SEQ ID NO: 6 (QHSRDLPLT). The pembrolizumab may include a heavy chain variable region having the amino acid sequence of SEQ ID NO: 7 (VQLVQSGVEVKKPGASVKVSCKASGYTFTNYYMYWVRQAPGQGLEWMGGINPS NGGTNFNEKFKNRVTLTTDSSTTTAYMELKSLQFDDTAVYYCARRDYRFDMGFDYWGQGTTVTVSSASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGK) and a light chain variable region having the amino acid sequence of SEQ ID NO: 8

(IVLTQSPATLSLSPGERATLSCRASKGVSTSGYSYLHWYQQKPGQAPRLLIYLASYL
ESGVPARFSGSGSGTDFTLTISSLEPEDFAVYYCQHSRDLPLTFGGGTKVEIKRTVA
APSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC).

The term "biosimilar" is also called an equivalent biopharmaceutical product. Biopharmaceuticals are not produced as chemical products but are produced through cells, and thus it is impossible to precisely copy an original drug, unlike a generic drug. Thus, a copied drug for biopharmaceuticals is termed a biosimilar because it is similar to but not exactly the same as the original drug.

The term "biobetter" refers to an improved drug over original biopharmaceuticals in terms of efficacy, safety, convenience, etc. In the sense that a follow-on drug is better than existing reference biopharmaceuticals, the follow-on drug is called a biobetter. Biobetters may be produced using bioengineering technologies, such as recombinant gene technology, cell culture technology.

The pembrolizumab targets a programmed cell death protein 1 (PD-1) receptor of a lymphocyte. The pembrolizumab is an IgG₄ iso-type antibody allowing the immune system to kill cancer cells by blocking the protection mechanism of cancer cells. The pembrolizumab was approved for medical use in the US in 2014. In addition, the pembrolizumab was approved for treatment of unresectable or metastatic solid tumors having certain genetic abnormalities, for example, mismatch repair deficiency, or microsatellite instability, in 2017. In addition, the pembrolizumab may be used alone or in combination with other chemical therapeutic agents. The pembrolizumab may be administered by intravenous or subcutaneous injection.

The pembrolizumab may be produced by a general method that is known in the related art. For example, US Patent 9,834,605 and WO2008/156712A1 disclose methods which those skilled in the art can use in producing a pembrolizumab. For example, the pembrolizumab may be produced by recombinant expression of immunoglobulin light and heavy chain genes in the host cell.

The anti-PD-1 antibody, for example, a pembrolizumab, or an antigen binding fragment thereof, has a pH buffering action in an aqueous solution. The concentration of the anti-PD-1 antibody, for example, a pembrolizumab, or the antigen binding fragment thereof, may be an amount suitable for providing a buffering action at a pH of 4.5 to 6.5. In addition, the concentration of the anti-PD-1 antibody, for example, a pembrolizumab, or the antigen binding fragment thereof, may be a therapeutically effective amount for treatment of a cancer. In addition, the concentration of the anti-PD-1 antibody, for example, a pembrolizumab, or the antigen binding fragment thereof, may be selected in consideration of the stability, viscosity, etc. of the pharmaceutical formulation. The concentration of the anti-PD-1 antibody, for example, a pembrolizumab, or the antigen binding fragment thereof, may be, for example, 5 to 300 mg/mL, 5 to 250 mg/mL, 5 to 200 mg/mL, 10 to 200 mg/mL, 10 to 165 mg/mL, 15 to 160 mg/mL, 5 to 45 mg/mL, 10 to 40 mg/mL, 15 to 35 mg/mL, 20 to 30 mg/mL, 130 to 250 mg/mL, 135 to 200 mg/mL, 135 to 170 mg/mL, 140 to 160 mg/mL, 145 to 155 mg/mL, about 25 mg/ml, about 150 mg/ml, about 200 mg/mL, or about 250 mg/mL. The anti-PD-1 antibody, for example, a pembrolizumab, or an antigen binding fragment thereof, may contribute to the stability of the pharmaceutical formulation and maintenance of the viscosity and pH suitable for administration at the concentrations listed above.

The term "stabilizer" means a material added to prevent a state change or a chemical change when a sample material is left undisturbed or preserved. The stabilizer may be one or more selected from the group consisting of polyols, amino acids, and metal salts.

As used herein, the term "polyol" refers to an excipient having multiple hydroxyl groups. The polyol may include a sugar, a sugar alcohol, or a sugar acid. The sugar refers to a soluble carbohydrate. The sugar may be a monosaccharide, a disaccharide, an oligosaccharide, or a polysaccharide. The sugar alcohol is an organic compound derived from sugar, and each carbon atom thereof has a hydroxyl group. The sugar acid refers to a sugar having a carboxyl group at one or both sides of the chain. The polyol may be one or more selected from the group consisting of glucose, fructose, mannose, galactose, sucrose, lactose, maltose, trehalose, mannitol, sorbitol, and polyethylene glycol. The polyol may be sorbitol, sucrose, trehalose, mannose, maltose, mannitol, or a mixture thereof. The polyol includes a polyol anhydride. For example, the trehalose may include trehalose dehydrate as well as trehalose. The concentration of the polyol may be freely adjusted within a desirable range for maintaining the stability of an anti-PD-1 antibody, for example, a pembrolizumab, or an antigen binding fragment thereof in the pharmaceutical formulation, and the viscosity of a liquid pharmaceutical formulation, and may individually vary depending on the specific type of various polyols, sugar alcohols or sugar acids.

The concentration of the sugar may be in a range of 1.0 to 15.0%(w/v), 3.0 to 15.0%(w/v), 5.0 to 15.0%(w/v), 7.0 to 15.0%(w/v), 1.0 to 10.0%(w/v), 3.0 to 10.0%(w/v), 4.0 to 10.0%(w/v), 5.0 to 10.0%(w/v), 7.0 to 10.0%(w/v), 1.0 to 7.0%(w/v), 2.0 to 7.0%(w/v), 3.0 to 7.0%(w/v), 4.0 to 7.0%(w/v), 1.0 to 5.0%(w/v), 2.0 to 5.0%(w/v), 3.0 to 5.0%(w/v), 4.0 to 5.0%(w/v), 4.5 to 5.0%(w/v) (e.g., about 4.7%(w/v)), 6.5 to 8.5%(w/v) (e.g., about 6.8%(w/v), about 7.0%(w/v), about 7.2%(w/v)), or 7.8 to 8.2%(w/v) (e.g., about 7.8%(w/v), about 7.9%(w/v), about 8.0%(w/v), about 8.1%(w/v), or about 8.2%(w/v)). The sugar may be, for example, sucrose, glucose, galactose, maltose, fructose, trehalose, or a mixture thereof. In a specific embodiment, the sugar may be 1.0 to 15.0%(w/v), 4.0 to 10.0%(w/v), 6.0 to 8.0%(w/v), or about 7.0%(w/v) of sucrose. In another specific embodiment, the sugar may be 1.0 to 15.0%(w/v), 4.0 to 10.0%(w/v), 7.0 to 8.0%(w/v), or about 7.6%(w/v) of trehalose.

The concentration of the sugar alcohol may be 1.0 to 20.0%(w/v), for example, 1.0 to 15.0%(w/v), 1.0 to 10.0%(w/v), 2.5 to 10.0%(w/v), 3.0 to 10.0%(w/v), 3.5 to 10.0%(w/v), 4.0 to 10.0%(w/v), 1.0 to 8.0%(w/v), 2.5 to 8.0%(w/v), 3.0 to 8.0%(w/v), 3.5 to 8.0%(w/v), 4.0 to 8.0%(w/v), 1.0 to 6.0%(w/v), 2.5 to 6.0%(w/v), 3.0 to 6.0%(w/v), 3.5 to 6.0%(w/v), 4.0 to 6.0%(w/v), 4.0 to 5.5%(w/v), or 4.0 to 5.0%(w/v). The sugar alcohol may be, for example, sorbitol, mannitol, a hydrate thereof, or a mixture thereof. In a specific embodiment, the sugar alcohol may be about 4.0%(w/v) of sorbitol.

The amino acid may be glycine, alanine, valine, leucine, isoleucine, proline, phenylalanine, tyrosine, tryptophan, serine, threonine, cysteine, methionine, asparagine, glutamine, lysine, arginine, histidine, aspartic acid, glutamic acid, a pharmaceutically acceptable salt thereof, or a mixture thereof. The amino acid may be, for example, glycine, proline, phenylalanine, tyrosine, tryptophan, lysine, arginine, a pharmaceutically acceptable salt thereof, or a mixture thereof. In a specific embodiment, the amino acid may be arginine, a pharmaceutically acceptable salt thereof, or a mixture thereof. In another specific embodiment, the amino acid may be proline, a pharmaceutically acceptable salt thereof, or a mixture thereof. In still another specific embodiment, the amino acid may be lysine, a pharmaceutically acceptable salt thereof, or a mixture thereof. In still another specific embodiment, the amino acid may be arginine, lysine, a pharmaceutically acceptable salt thereof, or a mixture thereof.

The concentration of the amino acid as the stabilizer may be in a range of 0.1 to 300.0 mM, 0.5 to 300.0 mM, 1.0 to 300.0 mM, 5.0 to 300.0 mM, 10.0 to 300.0 mM, 25.0 to 300.0 mM, 30.0 to 300.0 mM, 50.0 to 300.0 mM, 80.0 to 300.0 mM, 100.0 to 300.0 mM, 120.0 to 300.0 mM, 0.1 to 250.0 mM, 0.5 to 250.0 mM, 1.0 to 250.0 mM, 5.0 to 250.0 mM, 10.0 to 250.0 mM, 25.0 to 250.0 mM, 30.0 to 250.0 mM, 50.0 to 250.0 mM, 80.0 to 250.0 mM, 100.0 to 250.0 mM, 120.0 to 250.0 mM, 0.1 to 200.0 mM, 0.5 to 200.0 mM, 1.0 to 200.0 mM, 5.0 to 200.0 mM, 10.0 to 200.0 mM, 25.0 to 200.0 mM, 30.0 to 200.0 mM, 50.0 to 200.0 mM, 80.0 to 200.0 mM, 100.0 to 200.0 mM, 120.0 to 200.0 mM, 0.1 to 160.0 mM, 0.5 to 160.0 mM, 1.0 to 160.0 mM, 5.0 to 160.0 mM, 10.0 to 160.0 mM, 25.0 to 160.0 mM, 30.0 to 160.0 mM, 50.0 to 160.0 mM, 80.0 to 160.0 mM, 100.0 to 160.0 mM, 120.0 to 160.0 mM, 130.0 to 150.0 mM, 0.1 to 100.0 mM, 0.5 to 100.0 mM, 1.0 to 100.0 mM, 5.0 to 100.0 mM, 10.0 to 100.0 mM, 25.0 to 100.0 mM, 30.0 to 100.0 mM, 50.0 to 100.0 mM, 80.0 to 100.0 mM, 0.1 to 50.0 mM, 0.5 to 50.0 mM, 1.0 to 50.0 mM, 5.0 to 50.0 mM, 10.0 to 50.0 mM, 25.0 to 50.0 mM, 30.0 to 50.0 mM, 0.1 to 40.0 mM, 0.5 to 40.0 mM, 1.0 to 40.0 mM, 5.0 to 40.0 mM, 10.0 to 40.0 mM, 25.0 to 40.0 mM, 30.0 to 40.0 mM, 0.1 to 30.0 mM, 0.5 to 30.0 mM, 1.0 to 30.0 mM, 5.0 to 30.0 mM, 10.0 to 30.0 mM, 25.0 to 30.0 mM, 0.1 to 20.0 mM, 0.5 to 20.0 mM, 1.0 to 20.0 mM, 5.0 to 20.0 mM, 10.0 to 20.0 mM, 0.1 to 10.0 mM, 0.5 to 10.0 mM, 1.0 to 10.0 mM, or 5.0 to 10.0 mM. In a specific embodiment, the amino acid may be 1.0 to 10.0 mM, 1.0 to 5.0 mM, 2.0 to 10.0 mM, 2.0 to 5.0 mM, 2.0 to 4.0 mM, or about 3.3 mM of arginine, a pharmaceutically acceptable salt thereof, or a mixture thereof. In another specific embodiment, the amino acid may be 24 to 29 mM of glycine, a pharmaceutically acceptable salt thereof, or a mixture thereof. The concentration of the amino acid may be freely adjusted within the range in which the stability of an anti-PD-1 antibody, for example, a pembrolizumab, or an antigen binding fragment thereof, is not affected without affecting a desired pH of the pharmaceutical formulation, and may individually vary depending on the specific type of various amino acids.

The metal salt may be NaCl, KCI, NaF, KBr, NaBr, Na₂SO₄, NaSCN, CaCl₂, MgCl₂, or K₂SO₄. The metal salt may be, for example, NaCl or Na₂SO₄. The concentration of the metal salt may be 1.0 to 300.0 mM, 5.0 to 150.0 mM, 10.0 to 150.0 mM, 30.0 to 150.0 mM, 50.0 to 150.0 mM, 80.0 to 150.0 mM, 100.0 to 150.0 mM, 120.0 to 150.0 mM, 5.0 to 125.0 mM, 10.0 to 125.0 mM, 30.0 to 125.0 mM, 50.0 to 125.0 mM, 80.0 to 125.0 mM, 100.0 to 125.0 mM, 120.0 to 125.0 mM, 5.0 to 100.0 mM, 10.0 to 100.0 mM, 30.0 to 100.0 mM, 50.0 to 100.0 mM, 80.0 to 100.0 mM, 5 to 80.0 mM, 10.0 to 80.0 mM, 30.0 to 80.0 mM, or 50.0 to 80.0 mM. In a specific embodiment, the metal salt may be sodium chloride having a concentration of 5.0 to 150.0 mM, 20.0 to 140.0 mM, or about 100.0 mM. The concentration of the metal salt may be freely adjusted within the range in which the stability of an anti-PD-1 antibody in the pharmaceutical formulation according to the present disclosure, for example, a pembrolizumab or an antigen binding fragment thereof, is maintained without precipitation, and may individually vary depending on the specific type of various metal salts. The stabilizer may be a mixture of a polyol and an amino acid. When the mixture of a polyol and an amino acid is used, the amino acid may perform at least one function as a stabilizer or a viscosity reducing agent.

The phrase "the pharmaceutical formulation not comprising an ingredient A", as used herein may mean that the pharmaceutical formulation does not, or substantially not comprise, the ingredient A. The phrase "substantially not comprise an ingredient A" may be interpreted to encompass a case where the ingredient A is not present at all, a case where the ingredient A is present in a trace amount, if any, so as not to substantially affect features of the pharmaceutical formulation, or a case where the ingredient A is present in an undetectable amount.

The pharmaceutical formulation does not comprise any separate buffer other than the anti-PD-1 antibody, for example, a pembrolizumab, or the antigen binding fragment thereof. As used herein, the term "buffer" refers to a composition added to allow the pharmaceutical formulation to resist a change in the pH. The buffer may act to maintain a pH of the pharmaceutical formulation within a tolerable range. The buffer generally allows the pharmaceutical formulation to resist a change in the pH through actions of acid-base conjugate components thereof. In this specification, when a concentration of buffer is mentioned, the mentioned concentration refers to a molarity of the buffer in the form of a free acid or a free base thereof. In this specification, the phrase "not comprising a buffer" means that a buffer is not contained in an amount enough to allow the pharmaceutical formulation to resist a change in the pH. This may be interpreted to include an amount in which an intended function as a buffer cannot be exerted in the pharmaceutical formulation. The buffer may be histidine, phosphoric acid (sodium phosphorate or potassium phosphorate), maleic acid, tartaric acid, succinic acid (succinate), citric acid (citrate), acetic acid (acetate), carbonic acid, a pharmaceutically acceptable salt thereof, or a mixture thereof. In a specific embodiment, the buffer may be histidine, a pharmaceutically acceptable salt thereof, or a mixture thereof. In another specific embodiment, the buffer may be phosphoric acid, a pharmaceutically acceptable salt thereof, or a mixture thereof.

The pharmaceutical formulation may comprise, or may not comprise, a surfactant. The surfactant may be a non-ionic surfactant. The non-ionic surfactant may be polysorbate, poloxamer, sorbitan ester of another fatty acid, or a mixture thereof. The polysorbate may be polysorbate 20, polysorbate 40, polysorbate 60, polysorbate 80, or a mixture thereof. The poloxamer may be poloxamer 188. When the pharmaceutical formulation comprises a surfactant, the surfactant may have a concentration of 0.001 to 1%(w/v), 0.001 to 0.5%(w/v), 0.001 to 0.1%(w/v), 0.001 to 0.05%(w/v), 0.001 to 0.02%(w/v), 0.005 to 1%(w/v), 0.005 to 0.5%(w/v), 0.005 to 0.1%(w/v), 0.005 to 0.05%(w/v), 0.005 to 0.02%(w/v), 0.008 to 1%(w/v), 0.008 to 0.5%(w/v), 0.008 to 0.1%(w/v), 0.008 to 0.05%(w/v), or 0.008 to 0.02%(w/v), based on the total volume of the pharmaceutical formulation or a reconstituted formulation thereof. In a specific embodiment, the pharmaceutical formulation may comprise 0.02 to 0.04%(w/v) of polysorbate 20 or polysorbate 80.

The pharmaceutical formulation may further comprise an antioxidant. The term "antioxidant" means an oxidation resisting material. The antioxidant may be amino acid, vitamin, coenzyme, glutathione, methylsulfonylsulfate, or a mixture thereof. The amino acid as an antioxidant may be methionine, L-cysteine, L-carnitine, or a mixture thereof. The vitamin as an antioxidant may be vitamin A, vitamin C, vitamin E, or a mixture thereof. The coenzyme may be coenzyme Q10. In a specific embodiment, the antioxidant may be methionine.

In the pharmaceutical formulation, the concentration of the antioxidant may be freely adjusted within a desirable range for maintaining the stability of an anti-PD-1 antibody, for example, a pembrolizumab, or an antigen binding fragment thereof, and may individually vary depending on the specific type of various antioxidants. When an antioxidant is contained in the pharmaceutical formulation, the concentration of the antioxidant may be 1 to 70 mM, 1 to 50 mM, 1 to 30 mM, 5 to 50 mM, 5 to 30 mM, 1 to 20 mM, 1 to 15 mM, 1 to 10 mM, 1 to 5 mM, 3 to 20 mM, 3 to 15 mM, 3 to 10 mM, 3 to 7 mM, 20 to 60 mM, 30 to 50 mM, about 5 mM, about 20 mM, about 30 mM, about 40 mM, or about 50 mM. In a specific embodiment, the pharmaceutical formulation may comprise about 5 mM of methionine. In another specific embodiment, the pharmaceutical formulation may comprise about 30 mM of methionine. In still another specific embodiment, the pharmaceutical formulation may comprise about 50 mM of methionine.

The pharmaceutical formulation may be a liquid pharmaceutical formulation. The pharmaceutical formulation may be for subcutaneous or intravenous injection. The pharmaceutical formulation may further include an appropriate aqueous carrier suitable for injection. The aqueous carrier is a safe, non-toxic, pharmaceutically acceptable carrier that may be administered to a human, and examples thereof may include water, a saline solution, a Ringer's solution, dextrose, or a mixture thereof.

The pharmaceutical formulation may have an osmotic pressure being in an appropriate range for subcutaneous or intravenous injection. The osmotic pressure may be in a range of, for example, 200 to 400 mOsm/kg, 200 to 350 mOsm/kg, 250 to 300 mOsm/kg, 250 to 290 mOsm/kg, 270 to 328 mOsm/kg, 250 to 269 mOsm/kg, or 328 to 350 mOsm/kg. The osmotic pressure may be appropriately adjusted to minimize a pain that may be caused when the pharmaceutical formulation is administered.

The pharmaceutical formulation may have a viscosity in an appropriate range suitable for subcutaneous or intravenous injection. When a viscosity is measured at room temperature of 25°C±3°C, the viscosity may be, for example, 0.5 to 100 cp, 0.5 to 90 cp, 0.5 to 80 cp, 0.5 to 70 cp, 0.5 to 60 cp, 0.5 to 50 cp, 0.5 to 40 cp, 0.5 to 30 cp, 0.5 to 20 cp, 0.5 to 15 cp, or 0.5 to 10 cp. The viscosity may be appropriately adjusted to minimize a pain that may be caused when the pharmaceutical formulation is administered.

In the pharmaceutical formulation according to a specific embodiment, the anti-PD-1 antibody, for example, a pembrolizumab, or the antigen binding fragment thereof, may be contained in a concentration of 5 to 300 mg/mL, 5 to 250 mg/mL, 5 to 200 mg/mL, 15 to 30 mg/mL, or 150 to 250 mg/mL, the stabilizer may be sucrose, glucose, galactose, maltose, fructose, trehalose, sorbitol, mannitol, arginine, lysine, proline, glycine, phenylalanine, tyrosine, tryptophan, a hydrate thereof, a pharmaceutically acceptable salt thereof, a mixture thereof, or a metal salt, and the pharmaceutical formulation may have a pH in the range of 4.5 to 6.5, or 5.0 to 5.5.

In the specific embodiment, the stabilizer may be sucrose, glucose, galactose, maltose, fructose, trehalose, a hydrate thereof, or a mixture thereof, and the concentration thereof may be 1.0 to 15.0%(w/v), 3.0 to 15.0%(w/v), 5.0 to 15.0%(w/v), 7.0 to 15.0%(w/v), 1.0 to 10.0%(w/v), 3.0 to 10.0%(w/v), 5.0 to 10.0%(w/v), 7.0 to 10.0%(w/v), 1.0 to 7.0%(w/v), 2.0 to 7.0%(w/v), 3.0 to 7.0%(w/v), 4.0 to 7.0%(w/v), 1.0 to 5.0%(w/v), 2.0 to 5.0%(w/v), 3.0 to 5.0%(w/v), 4.0 to 5.0%(w/v), 4.5 to 5.0%(w/v) (e.g., about 4.7%(w/v)), or 7.8 to 8.2%(w/v) (e.g., about 7.8%(w/v), about 7.9%(w/v), about 8%(w/v), about 8.1%(w/v), or about 8.2%(w/v)). The stabilizer may be sorbitol, mannitol, a hydrate thereof, or a mixture thereof, and the concentration thereof may be 1.0 to 20.0%(w/v), for example, 1.0 to 15.0%(w/v), 1.0 to 10.0%(w/v), 2.5 to 10.0%(w/v), 3.0 to 10.0%(w/v), 3.5 to 10.0%(w/v), 4.0 to 10.0%(w/v), 1.0 to 8.0%(w/v), 2.5 to 8.0%(w/v), 3.0 to 8.0%(w/v), 3.5 to 8.0%(w/v), 4.0 to 8.0%(w/v), 1.0 to 6.0%(w/v), 2.5 to 6.0%(w/v), 3.0 to 6.0%(w/v), 3.5 to 6.0%(w/v), 4.0 to 6.0%(w/v), 4.0 to 5.5%(w/v), or 4.0 to 5.0%(w/v). The stabilizer may be arginine, lysine, proline, glycine, phenylalanine, tyrosine, tryptophan, a hydrate thereof, a pharmaceutically acceptable salt thereof, or a mixture thereof, and the concentration thereof may be 0.1 to 300.0 mM, 0.5 to 300.0 mM, 1.0 to 300.0 mM, 5.0 to 300.0 mM, 10.0 to 300.0 mM, 25.0 to 300.0 mM, 30.0 to 300.0 mM, 50.0 to 300.0 mM, 80.0 to 300.0 mM, 100.0 to 300.0 mM, 120.0 to 300.0 mM, 0.1 to 250.0 mM, 0.5 to 250.0 mM, 1.0 to 250.0 mM, 5.0 to 250.0 mM, 10.0 to 250.0 mM, 25.0 to 250.0 mM, 30.0 to 250.0 mM, 50.0 to 250.0 mM, 80.0 to 250.0 mM, 100.0 to 250.0 mM, 120.0 to 250.0 mM, 0.1 to 200.0 mM, 0.5 to 200.0 mM, 1.0 to 200.0 mM, 5.0 to 200.0 mM, 10.0 to 200.0 mM, 25.0 to 200.0 mM, 30.0 to 200.0 mM, 50.0 to 200.0 mM, 80.0 to 200.0 mM, 100.0 to 200.0 mM, 120.0 to 200.0 mM, 0.1 to 160.0 mM, 0.5 to 160.0 mM, 1.0 to 160.0 mM, 5.0 to 160.0 mM, 10.0 to 160.0 mM, 25.0 to 160.0 mM, 30.0 to 160.0 mM, 50.0 to 160.0 mM, 80.0 to 160.0 mM, 100.0 to 160.0 mM, 120.0 to 160.0 mM, 130.0 to 150.0 mM, 0.1 to 100.0 mM, 0.5 to 100.0 mM, 1.0 to 100.0 mM, 5.0 to 100.0 mM, 10.0 to 100.0 mM, 25.0 to 100.0 mM, 30.0 to 100.0 mM, 50.0 to 100.0 mM, 80.0 to 100.0 mM, 0.1 to 50.0 mM, 0.5 to 50.0 mM, 1.0 to 50.0 mM, 5.0 to 50.0 mM, 10.0 to 50.0 mM, 25.0 to 50.0 mM, 30.0 to 50.0 mM, 0.1 to 40.0 mM, 0.5 to 40.0 mM, 1.0 to 40.0 mM, 5.0 to 40.0 mM, 10.0 to 40.0 mM, 25.0 to 40.0 mM, 30.0 to 40.0 mM, 0.1 to 30.0 mM, 0.5 to 30.0 mM, 1.0 to 30.0 mM, 5.0 to 30.0 mM, 10.0 to 30.0 mM, 25.0 to 30.0 mM, 0.1 to 20.0 mM, 0.5 to 20.0 mM, 1.0 to 20.0 mM, 5.0 to 20.0 mM, 10.0 to 20.0 mM, 0.1 to 10.0 mM, 0.5 to 10.0 mM, 1.0 to 10.0 mM, or 5.0 to 10.0 mM. The stabilizer may be a mixture of: about 1.0 to about 15.0%(w/v) of sucrose, trehalose, a hydrate thereof, or a mixture thereof; and about 0.1 to about 300.0 mM of arginine, lysine, proline, glycine, phenylalanine, tyrosine, tryptophan, a pharmaceutically acceptable salt thereof, or a mixture thereof. The stabilizer may be a mixture of: about 1.0 to about 20.0%(w/v) of sorbitol, mannitol, a hydrate thereof, or a mixture thereof; and about 0.1 to about 300.0 mM of arginine, lysine, proline, glycine, phenylalanine, tyrosine, tryptophan, a pharmaceutically acceptable salt thereof, or a mixture thereof.

In the specific embodiment, the pharmaceutical formulation may further comprise a surfactant. The surfactant may be polysorbate or poloxamer. The polysorbate may be polysorbate 20 or polysorbate 80. The concentration of the surfactant may be 0.001 to 1%(w/v), 0.001 to 0.5%(w/v), 0.001 to 0.1%(w/v), 0.001 to 0.05%(w/v), 0.001 to 0.02%(w/v), 0.005 to 1%(w/v), 0.005 to 0.5%(w/v), 0.005 to 0.1%(w/v), 0.005 to 0.05%(w/v), 0.005 to 0.02%(w/v), 0.008 to 1%(w/v), 0.008 to 0.5%(w/v), 0.008 to 0.1%(w/v), 0.008 to 0.05%(w/v), 0.008 to 0.02%(w/v), or 0.02 to 0.04%(w/v).

In the specific embodiment, the pharmaceutical formulation may further comprise an antioxidant. The antioxidant may be methionine. The concentration of methionine may be 1 to 70 mM, 1 to 50 mM, 1 to 30 mM, 5 to 50 mM, 5 to 30 mM, 1 to 20 mM, 1 to 15 mM, 1 to 10 mM, 1 to 5 mM, 3 to 20 mM, 3 to 15 mM, 3 to 10 mM, 3 to 7 mM, 20 to 60 mM, 30 to 50 mM, about 5 mM, about 20 mM, about 30 mM, about 40 mM, or about 50 mM.

In the pharmaceutical formulation of the present disclosure, the anti-PD-1 antibody, for example, a pembrolizumab, or the antigen binding fragment thereof, may be stabilized. The term "stabilization" may mean that an anti-PD-1 antibody, for example, a pembrolizumab, or an antigen binding fragment thereof, substantially retains its physical stability, chemical stability and/or biological activity before and after administration, and during additional manufacturing processes, preservation or storage. The physical stability, chemical stability and/or biological activity may be evaluated by commonly known methods.

In this specification, the stability of an anti-PD-1 antibody or an antigen binding fragment thereof may meet one or more of the following items.

The stability may be measured at a selected temperature for a selected time period. For example, in a specific embodiment, a stable formulation is a formulation in which no significant change is observed at 2 to 8 °C for 12 months or more. In another specific embodiment, a stable formulation is a formulation in which no significant change is observed at 2 to 8 °C for 18 months or more. In another specific embodiment, a stable formulation is a formulation in which no significant change is observed at 23 to 27 °C for 3 months or more. In another specific embodiment, a stable formulation is a formulation in which no significant change is observed at 23 to 27 °C for 6 months or more. In another specific embodiment, a stable formulation is a formulation in which no significant change is observed at 23 to 27 °C for 12 months or more. In another specific embodiment, a stable formulation is a formulation in which no significant change is observed at 23 to 27 °C for 18 months or more. The followings are stability criteria for an antibody formulation. 10% or less, for example, 5% or less, or 2.5% or less of antibody monomers is denatured, as measured by SE-HPLC. The antibody has a potency within a range of 60 to 140%, or 80 to 120% of that of a control group or a standard antibody. For example, 10% or less, 5% or less, or 2.5% or less of antibody shows a change in the low molecular weight species (LMW), as measured by SE-HPLC. For example, 10% or less, 5% or less, or 2.5% or less of antibody shows a change in the high molecular weight species (HMW), as measured by SE-HPLC. In addition, the concentration and pH of the formulation may be changed in a range of ±10% or less, ±5% or less, or ±2.5% or less.

When the pharmaceutical formulation is placed in a stability thermostat and then stored under the conditions of 40±2 °C in temperature and 75±5% relative humidity for 4 weeks, the HMW or pH of the pharmaceutical formulation is changed within a range of 10% or less, 5% or less, or 2.5% or less.

When 0.3 to 1 mL of the pharmaceutical formulation is placed in a type I, 2 cc glass vial (Schott Inc.) and the vial is mounted at a stirrer (Heidolph Instruments) for stirring at room temperature at 400 rpm for 72 hours, the HMW or pH of the pharmaceutical formulation is changed within a range of 10% or less, 5% or less, or 2.5% or less.

The pembrolizumab or the antigen binding fragment thereof retains its chemical stability in the pharmaceutical formulation in a case where it is considered to be chemically stable at a predetermined time at which its biological activity is retained. The chemical stability may be evaluated by detecting chemically modified pembrolizumab and quantifying the same. The chemical modification includes size modification or charge change. The charge change may be a change resulting from, for example, deamidation. The size modification may be evaluated by size-exclusion high-performance liquid chromatography (SE-HPLC), sodium dodecyl sulfate-polyacrylamide gel (SDS-PAGE), capillary electrophoresis-sodium dodecyl sulfate (CE-SDS) assay, and matrix-assisted laser desorption/ionization/time-of-flight mass spectrometry (MALDI/TOF MS). In addition, the charge change may be evaluated by ion exchange chromatograph (IEC), and imaging capillary isoelectric focusing (icIEF). The activity may be determined by PD-1 ligand binding assay. The PD-1 ligand binding assay may be performed by enzyme-linked immunosorbent assay (ELISA). In the ELISA analysis, the relative binding rate (%) of pembrolizumab binding to PD-1 ligand is measured. A pembrolizumab sample is reacted with PD-1 ligand on ELISA plate, and the absorbance is measured at 450 nm to obtain a relative binding rate (%).

The anti-PD-1 antibody, for example, a pembrolizumab, or an antigen binding fragment thereof retains its biological activity in the pharmaceutical formulation. For example, when the biological activity of the anti-PD-1 antibody or the antigen binding fragment thereof in the pharmaceutical formulation is within about 30%, about 20%, or about 10% (or within an analysis error) at a manufacturing time point of the pharmaceutical formulation, the pharmaceutical formulation is considered to retain its biological activity. The biological activity may be determined by, for example, antigen binding assay.

The stability may be evaluated by measuring %HMW, %monomer and/or %LMW using size-exclusion HPLC (SE-HPLC) after applying temperature stress, for example, at 40 °C for 1 week to 4 weeks, freeze-thaw stress, for example, by repeating 5 times -70 °C freezing and room temperature thawing cycling, or stirring stress, for example, by applying a rotational force at 400 rpm for 72 hours. In a specific embodiment, Δ%HMW, Δ%LMW, or Δ% monomer values of the pharmaceutical formulation may be equal to or smaller than those of Keytruda^{®}. The stability may also be evaluated by measuring %acidic value variation using imaging capillary isoelectric focusing (icIEF) after applying temperature stress, freeze-thaw stress, or stirring stress. In a specific embodiment, the %acidic value of the pharmaceutical formulation may be equal to or smaller than those of Keytruda^{®}.

When the stable pharmaceutical formulation provided herein includes 25 mg/ml of an antibody (pH 5.5 or pH 5.0), a %HMW variation, that is, a difference between a %HMW value at week 4 and a %HMW value at week 0, may be 10.0% or less, 5.0% or less, or 2.5%, as measured by general SEC assay after 0.3 to 1 mL of the formulation is put into a type I, 2 cc glass vial (Schott Inc.) and then stored at 40 °C for 4 weeks.

When the stable pharmaceutical formulation provided herein includes 25 mg/ml of an antibody (pH 5.5 or pH 5.0), a %acidic value variation, that is, a difference between a %acidic value at week 4 and a %acidic value at week 0, may be 20.0% or less, 15.0% or less, or 10.0% or less, as measured by icIEF after the formulation is put into a polypropylene microtube, and then stored at 40 °C for 4 weeks.

In the specific embodiment, the pharmaceutical formulation may comprise: (i) an anti-PD-1 antibody or an antigen binding fragment thereof; (ii) a stabilizer; and (iii) a buffer except histidine, and may have a pH of about 4.5 to about 6.5. The buffer except histidine may be succinate, citrate, acetate, phosphate, or a combination thereof.

Another aspect provides a method for treating a cancer using the pharmaceutical formulation. The pharmaceutical formulation is the same as described above. The method for treating a cancer may comprising administering a therapeutically effective amount of the pharmaceutical formulation to a subject. The subject may be a human.

In the method, the term "cancer" refers to or describes pathological conditions in mammals, typically characterized by up-regulated cell growth. The cancer includes carcinoma, lymphoma, leukemia, blastoma and sarcoma, but not limited thereto. The cancer may include, for example, squamous cell carcinoma, myeloma, small cell lung cancer, non-small cell lung cancer, glioma, Hodgkin lymphoma, non-Hodgkin lymphoma, gastrointestinal (tract) cancer, renal cancer, ovarian cancer, liver cancer, lymphoblastic leukemia, lymphocytic leukemia, colorectal cancer, endometrial cancer, kidney cancer, prostate cancer, thyroid cancer, melanoma, chondrosarcoma, neuroblastoma, pancreatic cancer, glioblastoma multiforme, cervical cancer, brain cancer, stomach cancer, bladder cancer, hepatoma, breast cancer, colon carcinoma, Merkel cell cancer, microsatellite instability-high cancer, esophageal cancer, urothelial cancer, primary mediastinal large B-cell lymphoma, and head and neck cancer.

Another aspect provides a method for preparing a stable pharmaceutical formulation, comprising: preparing a mixed solution by adding a stabilizer to a solvent; and adding an anti-PD-1 antibody or an antigen binding fragment thereof to the mixed solution, or comprising: preparing a solution of an anti-PD-1 antibody or an antigen binding fragment thereof by adding the anti-PD-1 antibody or the antigen binding fragment thereof to a solvent; and adding a stabilizer to the solution, wherein the preparation method is performed without adding a buffer.

In the method, the solvent may be an aqueous solvent, for example, water or a saline solution. The method may further comprise adding a surfactant to the mixed solution having the anti-PD-1 antibody or the antigen binding fragment thereof added thereto. The method may further comprise adding an antioxidant to the mixed solution having the anti-PD-1 antibody or the antigen binding fragment thereof added thereto. The method may further comprise adjusting a pH of the formulation to about 4.5 to about 5.5, about 5.5, or about 5.0.

Among the terms or elements mentioned in the pharmaceutical formulation, the same terms or elements as those mentioned in the description of the claimed method for preparing the pharmaceutical formulation should be understood to be the same as those mentioned previously in the description of the claimed pharmaceutical formulation.

### ADVANTAGEOUS EFFECTS OF DISCLOSURE

In the stable anti-PD-1 antibody pharmaceutical formulation according to an aspect, an anti-PD-1 antibody can be stably retained even without having a buffer.

In the method for treating a cancer in a subject according to an aspect, the cancer can be efficiently treated in the subject.

In the method for preparing a stable anti-PD-1 antibody pharmaceutical formulation according to an aspect, the stable anti-PD-1 antibody pharmaceutical formulation can be efficiently prepared.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 shows Δ%HMW values of formulations including various buffers after applying temperature stress thereto for 4 weeks;
FIG. 2 shows the T_{agg} values of buffer-free formulations or formulations including different amounts of histidine buffers and surfactants;
FIG. 3 shows the ratios (348/332) depending on the change in denaturant concentrations measured in a buffer-free formulation and a formulation including 20 mM of histidine; and
FIG. 4 shows Δ%HMW values of formulations after applying temperature stress to the formulations including various amounts of buffers and PS80 surfactants for 4 weeks.

### MODE OF DISCLOSURE

Hereinafter, the present disclosure will be described in greater detail through the following examples. However, the following examples are provided only for illustration of the present disclosure, but the scope of the present disclosure is not limited by these examples.

### Materials and Methods

### 1. Size Exclusion Chromatography (SEC)

The purity of each sample was identified by size-exclusion chromatography. Percentages of antibody monomer, high molecular weight species (HMW), and low molecular weight species (LMW) are determined by the size-exclusion chromatography. In the size-exclusion chromatography, LMW is eluted later than HMW. The presence of HMW indicates formation of protein aggregates, and the presence of LMW indicates formation of protein fragments.

### 2. Dynamic Light Scattering (DLS) Assay

Aggregates in each sample were measured by dynamic light scattering assay. In detail, each sample was diluted using each buffer and loaded onto wells of a 96-well plate. The plate was loaded onto DynoPro^{®} Plate Reader^{™} II instrument (Wyatt Technology). The temperature of the sample was increased at a rate of 0.15 °C/min in a temperature range of 25 to 70 °C, and sizes of aggregates in the pharmaceutical formulation were measured by dynamic light scattering (DLS) assay. Evaluation of stability is performed to measure temperatures at a time point at which the aggregate size varies, and a higher T_{agg} value means a higher level of stability.

### 3. HUNKY (ΔG, AggPath) Assay

Aggregates in each sample were measured by HUNKY assay. HUNKY device (HUNKY, Unchained Labs.) is a device for identifying the stability of a protein through chemical denaturation, and enables C_{1/2}, ΔG, AggPath, and ΔGtrend values to be identified using denaturants and samples. C_{1/2} ranks stability. ΔG quantifies stability. AggPath predicts aggregation. ΔG_{trend} zooms in on aggregation.

### 4. Imaging Capillary Isoelectric Focusing (icIEF)

%acidic values of samples were measured using icIEF. Specifically, each sample was put into a 1.5 mL polypropylene microtube, and then microtube was exposed to a 40 °C temperature stress condition in a stability thermostat for 4 weeks.

The sample was injected into an iCE3 system (Protein Simple, USA), and charge variant values of proteins were measured using iCE CFR software. The charge variant values of proteins are indicated by %acidic values. Measurement of these values is for identifying charge variants of proteins. When a protein is denatured due to stress applied thereto, aggregation or a charge change thereof may occur. Therefore, the charge change of protein may serve as a factor for identifying the stability thereof.

### 5. Preparation of Pharmaceutical Formulations

Various pharmaceutical formulations listed in Table 1 were prepared in the following manners and used in the following examples.

First, in the composition of each of the respective pharmaceutical formulations, a buffer and a stabilizer, except for pembrolizumab and a surfactant, were added to sterilized distilled water to prepare a buffer solution. The pembrolizumab was introduced into a dialysis cassette (Slide-A-Lyzer cassette, Thermo Fisher Scientific) and then placed in a beaker containing a solution of each of the compositions listed in Table 1 to perform dialysis, thereby exchanging the existing pembrolizumab solution with the buffer solution. Finally, PS80 as a surfactant was added to the pembrolizumab solution such that the concentration of the surfactant in the prepared buffer solution reached a 1× concentration. Thereafter, the concentration was adjusted using each solution such that the pembrolizumab finally reached a concentration of 25 mg/mL.

**[Table 1]**

| Name | Group | Formulation composition |
|---|---|---|
| 1A | 1 (Buffer free) | Buffer free, 7% sucrose, pH 5.5 |
| 1B | | Buffer free, 7% sucrose, 0.02% PS80, pH 5.5 |
| 1C | | Buffer free, 7% sucrose, 0.10% PS80, pH 5.5 |
| 1D | | Buffer free, 7% sucrose, 0.20% PS80, pH 5.5 |
| 2A | 2 (histidine buffer) | 20 mM histidine, 7% sucrose, pH 5.5 |
| 2B | | 20 mM histidine, 7% sucrose, 0.02% PS80, pH 5.5 |
| 2C | | 20 mM histidine, 7% sucrose, 0.10% PS80, pH 5.5 |
| 2D | | 20 mM histidine, 7% sucrose, 0.20% PS80, pH 5.5 |
| 2E | | 40 mM histidine, 7% sucrose, pH 5.5 |
| 2F | | 40 mM histidine, 7% sucrose, 0.02% PS80, pH 5.5 |
| 2G | | 40 mM histidine, 7% sucrose, 0.10% PS80, pH 5.5 |
| 2H | | 40 mM histidine, 7% sucrose, 0.20% PS80, pH 5.5 |
| 2I | | 10 mM histidine, 7% sucrose, 0.02% PS80, pH 5.5 (Keytruda formulation) |
| 3A | 3 (acetic acid buffer) | 10 mM acetic acid, 7% sucrose, 0.20% PS80, pH 5.5 |
| 4A | 4 (succinic acid buffer) | 10 mM succinic acid, 7% sucrose, 0.20% PS80, pH 5.5 |
| 5A | 5 (citric acid buffer) | 10 mM citric acid, 7% sucrose, 0.20% PS80, pH 5.5 |

### Example 1: Effect of Buffer on Stability of Pembrolizumab-containing Pharmaceutical Formulation

In this example, the effect of a buffer on the stability of each of pembrolizumab-containing pharmaceutical formulations was identified. In detail, after applying temperature stress to formulations 1A, 21, 3A, 4A, and 5A, purities of the formulation samples were measured.

The temperature stress was applied such that each 0.3 mL of the formulations was put into a type I, 2 cc glass vial and the vial was exposed to a 40 °C temperature stress condition in a stability thermostat (JEIO TECH Co., Ltd.) for 4 weeks. Specifically, the vial was placed into the stability thermostat and stored under the conditions of 40±2 °C in temperature and 75±5% in relative humidity for 4 weeks. SEC analysis was performed on the formulations stored for 4 weeks in the above-described manner.

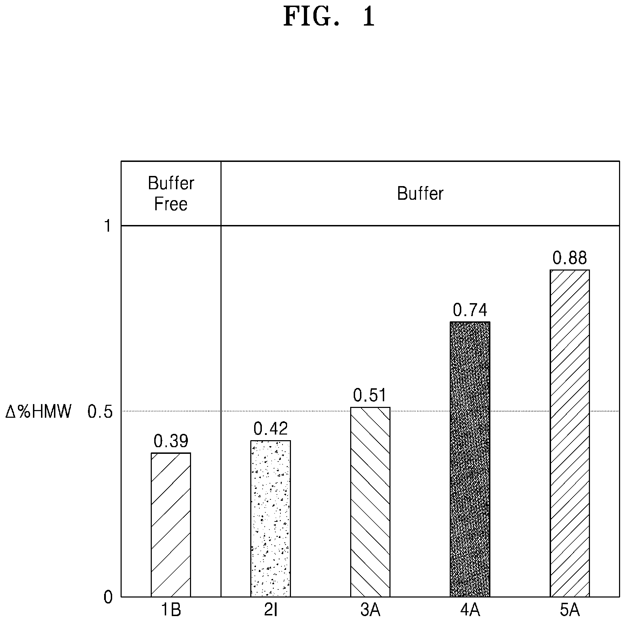

The results are shown in Table 2 and FIG. 1. Table 2 shows purities of formulations including various buffers and purities of the formulations after applying temperature stress thereto for 4 weeks, as indicated by %HMW. FIG. 1 shows Δ%HMW values of formulations including various buffers after applying temperature stress thereto for 4 weeks.

**[Table 2]**

| Name | 0 week | 4 weeks | |
|---|---|---|---|
| Name | %HMW | %HMW | Δ%HMW |
| 1B | 0.20 | 0.59 | 0.39 |
| 2I | 0.21 | 0.63 | 0.42 |
| 3A | 0.22 | 0.73 | 0.51 |
| 4A | 0.24 | 0.98 | 0.74 |
| 5A | 0.25 | 1.13 | 0.88 |

As shown in Table 2 and FIG. 1, at week 4, the average Δ%HMW value of the formulations each including a buffer and the Δ%HMW value of the buffer-free formulation were 0.64% and 0.39%, respectively, suggesting that the buffer-free formulation had higher stability than the formulations each including a buffer.

### Example 2: Effects of Amounts of Buffers and Surfactants on Stability of Pembrolizumab-containing Pharmaceutical Formulation

In this example, the effects of the amounts of buffers and surfactants on the stability of each of pembrolizumab-containing pharmaceutical formulations were identified. In detail, T_{agg} values of formulations 1A, 1B, 1C, 1D, 2A, 2B, 2C, 2D, 2E, 2F, 2G, and 2H were measured by DLS assay.

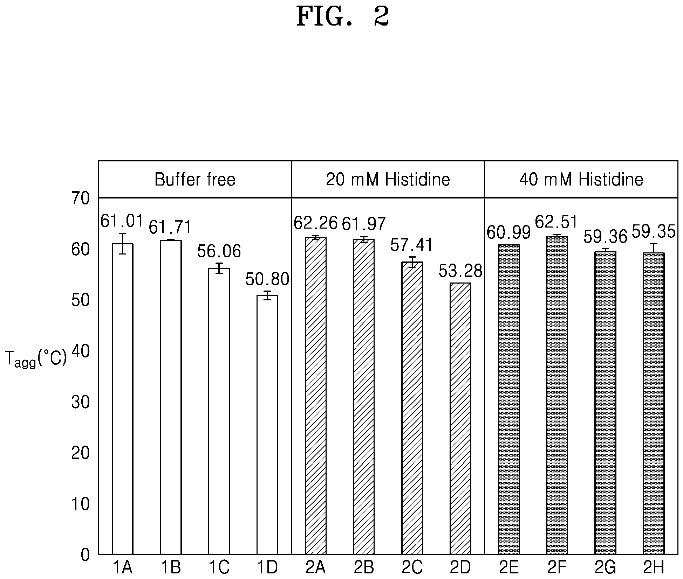

The results are shown in Table 3 and FIG. 2. Table 3 shows compositions of buffer-free formulations or formulations including histidine buffers and different amounts of PS80 surfactants, and T_{agg} values thereof. FIG. 2 shows the T_{agg} values of buffer-free formulations or formulations including histidine buffers and different amounts of surfactants.

**[Table 3]**

| Name | T_{agg} (°C) |
|---|---|
| 1A | 61.01 |
| 1B | 61.71 |
| 1C | 56.06 |
| 1D | 50.80 |
| 2A | 62.26 |
| 2B | 61.97 |
| 2C | 57.41 |
| 2D | 53.28 |
| 2E | 60.99 |
| 2F | 62.51 |
| 2G | 59.36 |
| 2H | 59.35 |

As shown in Table 3 and FIG. 2, T_{agg} values of the formulations not including PS80 or including 0.02% PS80 surfactants were higher than those of the formulations including 0.10% or 0.20% PS80 surfactants. This suggests that the formulations including less than 0.10% PS80 surfactants, that is, the formulations 1A, 1B, 2A, 2B, 2E, and 2F, had higher aggregation temperatures than the formulations including not less than 0.10% PS80 surfactants, that is, the formulations 1C, 1D, 2C, 2D, 2G, and 2H. That is, it is confirmed that the formulation including a low content, that is, less than 0.10%, of PS80, has higher stability than the formulation including a high content, that is, not less than 0.10%, of PS80.

Meanwhile, in the formulations 2A, 2B, 2E and 2F, each including less than 0.10% PS80, changes in the T_{agg} are not significantly affected by the amount of histidine.

In addition, among the formulations including 20 mM histidine buffers, the formulation not including PS80, that is, 2A, has a higher T_{agg} value than the formulations 2B, 2C, and 2D including PS80. By contrast, among the formulations including 40 mM histidine buffers, the formulation 2F including 0.02% PS80 has a higher T_{agg} value than the formulations 2E, 2G, and 2H.

### Example 3: Effects of Buffers on Stability of Pembrolizumab-containing Pharmaceutical Formulations

In this example, the effects of absence of buffer and 20 mM histidine buffer on the stability of each of the pembrolizumab-containing pharmaceutical formulations were identified. In detail, ΔG values of the formulations 1B and 2B were measured by HUNKY assay.

Unlike in the DLS assay for measuring protein aggregation depending on the temperature increase, in the HUNKY assay, the stability against chemical stress is measured using guanidine-HCI (Gdn-HCI). Specifically, in the HUNKY assay, an absorbance intensity ratio, that is, a ratio (348/332) is measured in a Gdn-HCI concentration gradient of 0 to 5.5 M at 348 nm and 332 nm. As a protein is denatured, the ratio increases.

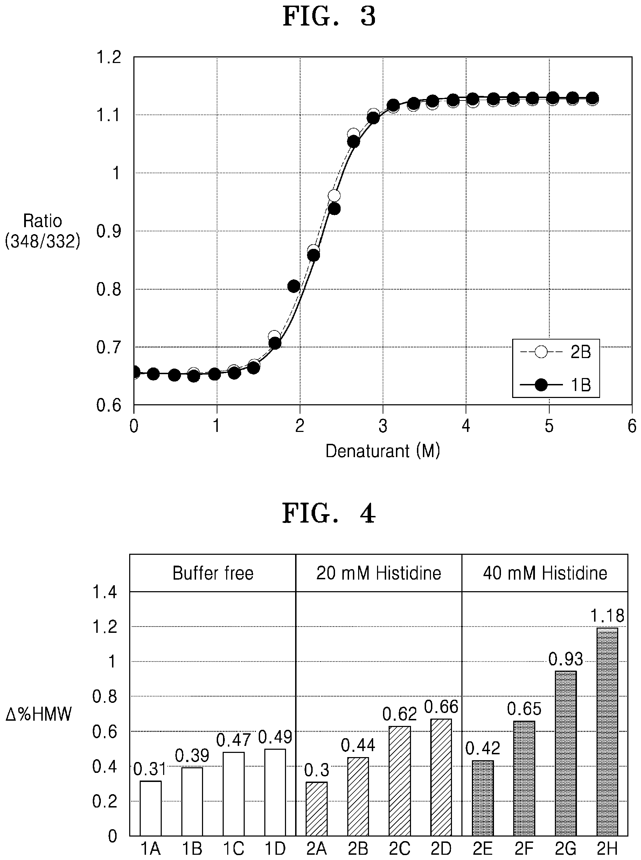

The results are shown in Table 4 and FIG. 3. Table 4 shows compositions of a buffer-free formulation and a formulation including 20 mM histidine, and ΔG values thereof. FIG. 3 shows the ratios (348/332) depending on the change in denaturant concentrations measured in a buffer-free formulation and a formulation including 20 mM histidine.

**[Table 4]**

| Name | ΔG1 (kcal/mol) |
|---|---|
| 1B | 4.26 |
| 2B | 4.50 |

As shown in Table 4 and FIG. 3, the ΔG values of the formulations 1B and 2B were equal levels. This suggests that the stability of the pembrolizumab-containing pharmaceutical formulation is not significantly affected by the presence of histidine buffer.

### Example 4: Effects of Amounts of Buffers and Surfactants on Stability of Pembrolizumab-containing Pharmaceutical Formulations

In this example, the effects of amounts of buffers and surfactants on the stability of each of the pembrolizumab-containing pharmaceutical formulations were identified. In detail, purities of formulation samples were measured by SEC after applying temperature stress to the formulations 1A, 1B, 1C, 1D, 2A, 2B, 2C, 2D, 2E, 2F, 2G, and 2H.

The temperature stress was applied such that each 0.3 mL of the formulations was put into a type I, 2 cc glass vial, and the vial was exposed to the 40 °C temperature stress condition in a stability thermostat for 4 weeks. Specifically, the vial was placed into the stability thermostat and stored under the conditions of 40±2 °C in temperature and 75±5% in relative humidity for 4 weeks. SEC analysis was performed on the formulations stored for 4 weeks in the above-described manner.

The results are shown in Table 5 and FIG. 4. Table 5 shows compositions of formulations including various amounts of buffers and PS80 surfactants and purities of the formulations after applying temperature stress thereto for 4 weeks, as indicated by %HMW. FIG. 4 shows Δ%HMW values of formulations including various amounts of buffers and PS80 surfactants after applying temperature stress thereto for 4 weeks.

**[Table 5]**

| Name | 0 week | 4 weeks | |
|---|---|---|---|
| | % HMW | % HMW | Δ% HMW |
| 1A | 0.19 | 0.50 | 0.31 |
| 1B | 0.20 | 0.59 | 0.39 |
| 1C | 0.28 | 0.75 | 0.47 |
| 1D | 0.41 | 0.90 | 0.49 |
| 2A | 0.18 | 0.48 | 0.30 |
| 2B | 0.18 | 0.62 | 0.44 |
| 2C | 0.25 | 0.87 | 0.62 |
| 2D | 0.39 | 1.05 | 0.66 |
| 2E | 0.18 | 0.60 | 0.42 |
| 2F | 0.19 | 0.84 | 0.65 |
| 2G | 0.27 | 1.20 | 0.93 |
| 2H | 0.41 | 1.59 | 1.18 |

As shown in Table 5 and FIG. 4, at week 4, the average Δ%HMW value of the formulations each including 20 mM histidine buffer and the Δ%HMW value of the buffer-free formulation were 0.51% and 0.42%, respectively, suggesting that the buffer-free formulation had higher stability than the formulations each including 20 mM histidine buffer. By contrast, the average Δ%HMW value of the formulations each including 40 mM histidine buffer was 0.80%, meaning that the stability was lowest.

In addition, among the formulations including 20 mM histidine buffers, the formulation not including PS80, that is, 2A, had a lower Δ%HMW value than the formulations each including PS80, that is, 2B, 2C and 2D. In addition, among the formulations including 40 mM histidine buffers, the formulation not including PS80, that is, 3A, has a lower Δ%HMW value than the formulations each including PS80, that is, 3B, 3C and 3D. This suggests that the formulations each free of buffer and PS80 have higher stability than the formulations each including buffer and PS80.

For the formulations stored under stress conditions for 4 weeks, pH values thereof were also measured. Table 6 shows pH measurement results of the samples left under 40 °C temperature stress conditions for 4 weeks.

**[Table 6]**

| Name | pH | |
|---|---|---|
| | 40 °C/0 week | 40 °C/4 weeks |
| 1A | 5.50 | 5.57 |
| 1B | 5.47 | 5.60 |
| 1C | 5.46 | 5.53 |
| 1D | 5.49 | 5.55 |
| 2A | 5.59 | 5.55 |
| 2B | 5.57 | 5.53 |
| 2C | 5.58 | 5.54 |
| 2D | 5.64 | 5.52 |
| 2E | 5.56 | 5.52 |
| 2F | 5.54 | 5.51 |
| 2G | 5.55 | 5.50 |
| 2H | 5.53 | 5.51 |

As shown in Table 6, the formulations including 20 mM histidine buffers and 40 mM histidine buffers and the buffer-free formulations showed no significant difference in the pH variation under 40 °C/4 week stress conditions, compared to initial pH values. It was confirmed that a pH buffering action was exerted in the formulation not including a buffer, like in the formulation including a buffer.

### Example 5: Effects of Buffer and pH Change on Stability of Pembrolizumab-containing Pharmaceutical Formulation

In this example, the effects of presence of buffer and pH change on the stability of each of the pembrolizumab-containing pharmaceutical formulations were identified.

First, the respective pharmaceutical formulations listed in Table 7 were prepared in the same manner as described above.

**[Table 7]**

| Name | Pembrolizumab | Buffer | Stabilizer | Surfactant | pH |
|---|---|---|---|---|---|
| 2I | 25 mg/ml | 10 mM histidine | 7% sucrose | 0.02% PS80 | 5.5 |
| 6A | 25 mg/ml | - | 7% sucrose | 0.02% PS80 | 5.5 |
| 6B | 25 mg/ml | 10 mM histidine | 7% sucrose | 0.02% PS80 | 5.0 |
| 6C | 25 mg/ml | - | 7% sucrose | 0.02% PS80 | 5.0 |

Purities of the formulations were measured by performing SEC analysis after applying temperature stress to the respective formulations in the same manner as in Example 1.

The results are shown in Table 8. Table 8 shows purities of the buffer-free formulations and formulations including a buffer and having pH variations, as indicated by %HMW.

**[Table 8]**

| Name | Composition | 0 week |
|---|---|---|
| | | %HMW |
| 2I | 10 mM histidine, 7% sucrose, 0.02% PS80, pH 5.5 | 0.34 |
| 6A | buffer free, 7% sucrose, 0.02% PS80, pH 5.5 | 0.18 |
| 6B | 10 mM histidine, 7% sucrose, 0.02% PS80, pH 5.0 | 0.16 |
| 6C | buffer free, 7% sucrose, 0.02% PS80, pH 5.0 | 0.14 |

As shown in Table 8, the buffer-free formulations had lower %HMW values than the formulations each including a buffer. In addition, the %HMW value of the formulation having a pH 5.0 was lower than that of the formulation having a pH 5.5. Specifically, the %HMW value of the buffer-free formulation having pH 5.0 was lowest. Accordingly, it was confirmed that the buffer-free formulation had higher stability than the formulation including a buffer, the formulation having pH 5.0 had higher stability than the formulation having pH 5.5, and the stability of the buffer-free formulation having pH 5.0 was highest.

In addition, %acidic values of the formulations were measured in the same manner as described above, using icIEF, after applying temperature stress to the respective formulations listed in Table 7.

The results are shown in Table 9. Table 9 shows %acidic values of buffer-free formulations and formulations including a buffer and having various pH levels.

**[Table 9]**

| Name | Temperature stress (40 °C): icIEF |
|---|---|
| | 0 week |
| | %acidic |
| 2I | 23.90 |
| 6A | 20.51 |
| 6B | 20.38 |
| 6C | 20.41 |

As shown in Table 9, the buffer-free formulations had lower %acidic values than the formulations each including a buffer. In addition, the %acidic value of the formulation having pH 5.0 was lower than that of the formulation having pH 5.5. Specifically, the %acidic value of the buffer-free formulation having pH 5.0 was lowest. Accordingly, it was confirmed that the buffer-free formulation had higher stability than the formulation including a buffer, the formulation having pH 5.0 had higher stability than the formulation having pH 5.5, and the stability of the buffer-free formulation having pH 5.0 was highest.

### Example 6: Extension of Stabilizers

In this example, the effects of changes in kinds of stabilizers and addition of an antioxidant on the stability of each of the pembrolizumab-containing pharmaceutical formulations were identified.

First, the respective pharmaceutical formulations listed in Table 10 were prepared in the same manner as described above.

**[Table 10]**

| Name | Pembro lizumab | Buffer | Stabilizer | | Surfactant | Antioxidant | pH |
|---|---|---|---|---|---|---|---|
| 7A | 25 mg/ml | - | 7% sucrose | 3.3 mM Arg | 0.02% PS80 | 5.0 mM Met | 5.0 |
| 7B | 25 mg/ml | - | 4% sorbitol | 3.3 mM Arg | 0.02% PS80 | 5.0 mM Met | 5.0 |
| 7C | 25 mg/ml | - | 7.6% trehalose | 3.3 mM Arg | 0.02% PS80 | 5.0 mM Met | 5.0 |
| 6A | 25 mg/ml | 10 mM histidine | 7% sucrose | - | 0.02% PS80 | - | 5.5 |

Purities of the respective formulations listed in Table 10 were measured by performing SEC analysis after applying temperature stress to the respective formulations in the same manner as in Example 1.

The results are shown in Table 11. Table 11 shows purities of the formulations including various kinds of stabilizers and having an antioxidant added thereto, as indicated by %HMW.

**[Table 11]**

| Name | 0 week | 1 week | | 2 weeks | |
|---|---|---|---|---|---|
| | %HMW | %HMW | Δ%HMW | %HMW | Δ%HMW |
| 7A | 0.32 | ND | ND | 0.64 | 0.32 |
| 7B | 0.17 | 0.43 | 0.26 | 0.55 | 0.38 |
| 7C | 0.19 | 0.40 | 0.21 | 0.54 | 0.35 |
| 6A | 0.36 | ND | ND | 0.76 | 0.40 |

| | | | | | |
|---|---|---|---|---|---|
| * ND: Not determined | | | | | |

As shown in Table 11, the buffer-free formulations each having an antioxidant added thereto and having a pH 5.0, that is, formulations 7A, 7B, and 7C, had lower Δ %HMW values than the formulation including a buffer, not including an antioxidant, and having a pH 5.5, that is, the formulation 6A. That is, the buffer-free formulation having an antioxidant added thereto and having a pH 5.0 had higher stability than the formulation including a buffer, not including an antioxidant, and having a pH of 5.5.

In addition, the buffer-free formulations each having an antioxidant added thereto and having a pH of 5.0 showed excellent stability even when the kinds of stabilizers were variously changed to sucrose, sorbitol, trehalose, arginine, or a combination thereof.

In addition, %acidic values of the respective formulations indicated in Table 10 were measured in the same manner as described above, using icIEF, after applying temperature stress to the respective formulations.

The results are shown in Table 12. Table 12 shows %acidic values of the formulations including various kinds of stabilizers and having antioxidant added thereto.

**[Table 12]**

| Name | Temperature stress (40°C): icIEF | | | | | | |
|---|---|---|---|---|---|---|---|
| | 0 week | 1 week | | 2 weeks | | 4 weeks | |
| | %acidic | %acidi c | Δ%acidi c | %acidic | Δ%acidi c | %acidic | Δ%acidi c |
| 7A | 22.48 | ND | ND | 24.58 | 2.10 | 29.42 | 6.95 |
| 7B | 20.57 | 21.95 | 1.38 | 24.08 | 3.51 | ND | ND |
| 7C | 20.97 | 21.95 | 0.98 | 24.05 | 3.08 | ND | ND |
| 6A | 22.34 | ND | ND | 24.92 | 2.58 | 30.78 | 8.44 |

| | | | | | | | |
|---|---|---|---|---|---|---|---|
| * ND: Not determined | | | | | | | |

As shown in Table 12, the buffer-free formulations each having an antioxidant added thereto and having a pH of 5.0, that is, formulations 7A, 7B, and 7C, had Δ%acidic values similar to or lower than the Δ%acidic value of the formulation including a buffer, not including an antioxidant, and having a pH of 5.5, that is, the formulation 6A. specifically, the Δ%acidic value of the formulation 7A was lowest, suggesting that the formulation 7A had highest stability.

### Example 7: Effect of Kind of Buffer on Stability of Pembrolizumab-containing Pharmaceutical Formulation

In this example, the effect of the kind of buffer on the stability of each of the pembrolizumab-containing pharmaceutical formulations was identified.

First, the respective pharmaceutical formulations listed in Table 13 were prepared in the same manner as described above.

**[Table 13]**

| Name | Pembrolizumab | Buffer | Stabilizer | Surfactant | pH |
|---|---|---|---|---|---|
| 8A | 25 mg/ml | 10 mM acetate | 128 mM Arg-HCI | 0.02% PS80 | 5.5 |
| 8B | 25 mg/ml | 10 mM acetate | 0.7% NaCl | 0.02% PS80 | 5.5 |
| 8C | 25 mg/ml | 10 mM histidine | 128 mM Arg-HCI | 0.02% PS80 | 5.5 |
| 8D | 25 mg/ml | 10 mM histidine | 0.7% NaCl | 0.02% PS80 | 5.5 |
| 8E | 25 mg/ml | 10 mM citrate | 128 mM Arg-HCI | 0.02% PS80 | 5.5 |
| 8F | 25 mg/ml | 10 mM citrate | 0.7% NaCl | 0.02% PS80 | 5.5 |
| 8G | 25 mg/ml | 10 mM succinate | 128 mM Arg-HCI | 0.02% PS80 | 5.5 |
| 8H | 25 mg/ml | 10 mM succinate | 0.7% NaCl | 0.02% PS80 | 5.5 |

Purities of the formulation samples of Table 13 were measured by performing SEC analysis after applying temperature stress to the respective formulations in the same manner as in Example 1.

The results are shown in Table 14. Table 14 shows purities of the formulations having different kinds of buffers, as indicated by %HMW.

**[Table 14]**

| Name | 0 week | 1 week | | 2 weeks | | 4 weeks | |
|---|---|---|---|---|---|---|---|
| | %HMW | %HMW | Δ%HMW | %HMW | Δ%HMW | %HMW | Δ%HMW |
| 8A | 0.25 | 0.85 | 0.60 | 1.23 | 0.98 | 1.90 | 1.65 |
| 8B | 0.27 | 0.90 | 0.63 | 1.27 | 1.00 | 1.93 | 1.66 |
| 8C | 0.25 | 0.90 | 0.65 | 1.32 | 1.07 | 2.02 | 1.77 |
| 8D | 0.26 | 0.83 | 0.57 | 1.21 | 0.95 | 1.86 | 1.60 |
| 8E | 0.24 | 0.75 | 0.51 | 1.07 | 0.83 | 1.71 | 1.47 |
| 8F | 0.28 | 0.82 | 0.54 | 1.11 | 0.83 | 1.65 | 1.37 |
| 8G | 0.26 | 0.73 | 0.47 | 1.05 | 0.79 | 1.76 | 1.50 |
| 8H | 0.28 | 0.77 | 0.49 | 1.10 | 0.82 | 1.71 | 1.43 |

As shown in Table 14, the formulations each including histidine, that is, 8C and 8D, had higher Δ%HMW values than the formulation including other kinds of buffers. Therefore, it was confirmed that the formulations each including histidine were lower stability than the formulation including other kinds of buffers.

### Example 8: Stabilities of High-Content Pembrolizumab-Containing Pharmaceutical Formulations

In this example, the stability of each of high-content pembrolizumab-containing pharmaceutical formulations was identified.

First, the pharmaceutical formulations listed in Table 15 were prepared in the same manner as described above.

**[Table 15]**

| Name | Pembrolizumab | Buffer | Stabilizer | | | Surfactant | Antioxidant | pH |
|---|---|---|---|---|---|---|---|---|
| 9A | 150 mg/ml | - | 7% sucrose | 10 mM Arg | 50 mM Lys | 0.02% PS80 | 30 mM Met | 5.5 |
| 9B | 150 mg/ml | - | 1% sucrose | 50 mM Arg | 50 mM Lys | 0.02% PS80 | 30 mM Met | 5.5 |
| 9C | 150 mg/ml | - | 7% sucrose | 50 mM Arg | - | 0.02% PS80 | 30 mM Met | 5.5 |
| 9D | 150 mg/ml | - | 7% sucrose | 20 mM Arg | - | 0.02% PS80 | 30 mM Met | 5.0 |
| 9E | 150 mg/ml | - | - | 20 mM Arg | - | 0.02% PS80 | - | 5.0 |
| 9F | 150 mg/ml | - | 5% sucrose | 60 mM Arg | - | 0.02% PS80 | 30 mM Met | 5.0 |
| 9G | 150 mg/ml | - | 7% sucrose | 30 mM Pro | - | 0.02% PS80 | 30 mM Met | 5.0 |
| 9H | 150 mg/ml | - | 7% sucrose | 20 mM Lys | - | 0.02% PS80 | 30 mM Met | 5.0 |
| 9I | 150 mg/ml | - | 7% sucrose | 20 mM Arg | - | 0.02% PS80 | 30 mM Met | 5.0 |
| 9J | 150 mg/ml | - | 7% sucrose | 20 mM Lys | - | 0.02% PS80 | 30 mM Met | 5.0 |
| 9K | 150 mg/ml | 10 mM histidine | 7% sucrose | - | - | 0.02% PS80 | - | 5.5 |

Purities of formulations 9A to 9D and 9K, among the formulation samples of Table 15, were measured by performing SEC analysis after applying temperature stress to the formulations 9A to 9D and 9K in the same manner as in Example 1. The results are shown in Table 16.

**[Table 16]**

| Name | 0 week | 1 week | | 2 weeks | |
|---|---|---|---|---|---|
| | %HMW | %HMW | Δ%HMW | %HMW | Δ%HMW |
| 9A | 0.50 | 1.35 | 0.85 | 1.70 | 1.20 |
| 9B | 0.44 | 1.58 | 1.14 | 2.03 | 1.59 |
| 9C | 0.49 | 1.35 | 0.86 | 1.70 | 1.21 |
| 9D | 0.43 | 1.63 | 1.20 | 2.13 | 1.70 |
| 9K | 0.64 | ND | ND | 2.35 | 1.71 |

| | | | | | |
|---|---|---|---|---|---|
| * ND: Not determined | | | | | |

As shown in Table 16, the formulations 9A to 9D each including a high content, that is, 150 mg/ml, of pembrolizumab, and not including a buffer, showed higher stability than the formulation including a buffer, that is, the formulation 9K.

In addition, %acidic values of the respective formulations indicated in Table 15 were measured in the same manner as described above, using icIEF, after applying temperature stress to the respective formulations. The results are shown in Table 17.

**[Table 17]**

| Name | Temperature stress (40°C): icIEF | | | | | | |
|---|---|---|---|---|---|---|---|
| | 0 week | 1 week | | 2 weeks | | 4 weeks | |
| | %acidic | %acidic | Δ%acidic | %acidic | Δ%acidic | %acidic | Δ%acidic |
| 9A | 20.74 | 21.93 | 1.19 | 23.84 | 3.10 | ND | ND |
| 9B | 20.72 | 21.87 | 1.15 | 23.68 | 2.96 | ND | ND |
| 9C | 20.42 | 21.76 | 1.34 | 23.61 | 3.19 | ND | ND |
| 9D | 20.96 | 22.05 | 1.09 | 24.29 | 3.33 | ND | ND |
| 9E | 22.86 | 23.34 | 0.48 | 24.89 | 2.03 | 28.86 | 6.00 |
| 9F | 22.44 | 23.26 | 0.82 | 25.00 | 2.56 | 28.41 | 5.97 |
| 9G | 22.80 | 23.86 | 1.06 | 25.64 | 2.84 | 29.99 | 7.19 |
| 9H | 22.92 | 23.34 | 0.42 | 25.16 | 2.24 | 28.00 | 5.08 |
| 9I | 20.02 | 21.52 | 1.50 | 24.19 | 4.17 | 28.86 | 8.84 |
| 9J | 20.21 | 20.55 | 0.34 | 23.83 | 3.62 | 28.86 | 8.65 |
| 9K | 24.6 | ND | ND | 28.9 | 4.3 | 36.69 | 12.09 |

| | | | | | | | |
|---|---|---|---|---|---|---|---|
| * ND: Not determined | | | | | | | |

As shown in Table 17, the formulations 9A to 9J each including a high content, that is, 150 mg/ml, of pembrolizumab, and not including a buffer, showed higher stability than the formulation including a buffer, that is, the formulation 9K.

In addition, the respective pharmaceutical formulations listed in Table 18 were prepared in the same manner as described above.

**[Table 18]**

| Name | Pembrolizumab | Buffer | Stabilizer | | | Surfactant | Antioxidant | pH |
|---|---|---|---|---|---|---|---|---|
| 10A | 200 mg/ml | - | 5% sucrose | 80 mM Arg | - | 0.02% PS80 | 30 mM Met | 5.0 |
| 10B | 200 mg/ml | - | 7% sucrose | 40 mM Lys | - | 0.02% PS80 | 30 mM Met | 5.0 |
| 10C | 200 mg/ml | | 7% sucrose | 26.4 mM Arg | - | 0.02% PS80 | 40 mM Met | 5.0 |
| 10D | 200 mg/ml | - | 7% sucrose | 13.2mM Arg | 13.2 mM Lys | 0.02% PS80 | 40 mM Met | 5.0 |
| 10E | 200 mg/ml | - | 4% sorbitol | 13.2mM Arg | 13.2 mM Lys | 0.02% PS80 | 40 mM Met | 5.0 |
| 10F | 250 mg/ml | - | 7% sucrose | 33.3 mM Arg | - | 0.02% PS80 | 50 mM Met | 5.0 |
| 10G | 250 mg/ml | - | 7% sucrose | 33.3 mM Lys | - | 0.02% PS80 | 50 mM Met | 5.0 |
| 10H | 250 mg/ml | - | 7% sucrose | 16.7 mM Arg | 16.7 mM Lys | 0.02% PS80 | 50 mM Met | 5.0 |
| 101 | 250 mg/ml | - | 4% sorbitol | 16.7 mM Arg | 16.7 mM Lys | 0.02% PS80 | 50 mM Met | 5.0 |

%acidic values of the respective formulations indicated in Table 18 were measured in the same manner as described above, using icIEF, after applying temperature stress to the respective formulations. The results are shown in Table 19.

**[Table 19]**

| Name | 0 week | 4 weeks | |
|---|---|---|---|
| | %acidic | %acidic | Δ%acidic |
| 10A | 22.19 | 28.73 | 6.54 |
| 10B | 22.66 | 28.86 | 6.20 |
| 10C | 19.72 | 28.86 | 9.14 |
| 10D | 19.41 | 28.86 | 9.45 |
| 10E | 20.01 | 28.86 | 8.85 |
| 10F | 19.76 | 28.86 | 9.10 |
| 10G | 19.56 | 28.86 | 9.30 |
| 10H | 19.76 | 28.86 | 9.10 |
| 10I | 19.92 | 28.86 | 8.94 |

As shown in Table 19, the buffer-free formulations 10A to 10I each including a high content, that is, 200 mg/ml or 250 mg/ml, of pembrolizumab, and not including a buffer, showed higher stability than the formulation including a buffer, that is, the formulation 9K.

## Claims

1. A stable anti-PD-1 antibody pharmaceutical formulation, comprising:
(a) an anti-PD-1 antibody or an antigen binding fragment thereof; and
(b) a stabilizer,
wherein the formulation does not comprise a buffer and has a pH of about 4.5 to about 6.5.

2. The pharmaceutical formulation of claim 1, wherein the formulation has a pH of about 5.0 to about 5.5.

3. The pharmaceutical formulation of claim 1, wherein the anti-PD-1 antibody includes a heavy chain CDR1 having the amino acid sequence of SEQ ID NO: 1, a heavy chain CDR2 having the amino acid sequence of SEQ ID NO: 2, and a heavy chain CDR3 having the amino acid sequence of SEQ ID NO: 3; and a light chain CDR1 having the amino acid sequence of SEQ ID NO: 4, a light chain CDR2 having the amino acid sequence of SEQ ID NO: 5, and a light chain CDR3 having the amino acid sequence of SEQ ID NO: 6.

4. The pharmaceutical formulation of claim 1, wherein the anti-PD-1 antibody is a pembrolizumab.

5. The pharmaceutical formulation of claim 1, wherein the concentration of the anti-PD-1 antibody or the antigen binding fragment thereof is about 5 to about 200 mg/ml.

6. The pharmaceutical formulation of claim 1, wherein the stabilizer is a polyol, an amino acid or a pharmaceutically acceptable salt, or a mixture thereof.

7. The pharmaceutical formulation of claim 6, wherein the polyol is sorbitol, sucrose, trehalose, mannose, maltose, mannitol, or a mixture thereof.

8. The pharmaceutical formulation of claim 6, wherein the amino acid is glycine, proline, phenylalanine, tyrosine, tryptophan, lysine, arginine, a pharmaceutically acceptable salt thereof, or a mixture thereof.

9. The pharmaceutical formulation of claim 1, wherein the stabilizer is about 1.0 to about 15.0%(w/v) of a sugar, about 1.0 to about 20.0%(w/v) of a sugar alcohol, about 0.1 to about 300.0 mM of an amino acid, or about 1.0 to about 300.0 mM of a metal salt.

10. The pharmaceutical formulation of claim 1, wherein the stabilizer is about 1.0 to about 15.0%(w/v) of a sugar and about 0.1 to about 300.0 mM of an amino acid.

11. The pharmaceutical formulation of claim 1, wherein the buffer is histidine, phosphoric acid, maleic acid, tartaric acid, succinic acid, citric acid, acetic acid, carbonic acid, a pharmaceutically acceptable salt thereof, or a mixture thereof.

12. The pharmaceutical formulation of claim 1, wherein the pharmaceutical formulation does not comprise a surfactant or further comprises a surfactant.

13. The pharmaceutical formulation of claim 12, wherein the surfactant is polysorbate, poloxamer, sorbitan ester of another fatty acid, or a mixture thereof.

14. The pharmaceutical formulation of claim 1, further comprising an antioxidant.

15. The pharmaceutical formulation of claim 14, wherein the antioxidant is methionine.

16. The pharmaceutical formulation of claim 1, wherein the pharmaceutical formulation is for administration by intravenous or subcutaneous injection.

17. The pharmaceutical formulation of claim 1, wherein the concentration of the anti-PD-1 antibody or the antigen binding fragment thereof is about 5 to about 200 mg/mL, and the stabilizer is: about 1.0 to about 15.0%(w/v) of sucrose, trehalose, a hydrate thereof, or a mixture thereof; about 1.0 to about 20.0 %(w/v) of sorbitol, mannitol, a hydrate thereof, or a mixture thereof; about 0.1 to about 300.0 mM of arginine, lysine, proline, glycine, phenylalanine, tyrosine, tryptophan, a pharmaceutically acceptable salt thereof; or a mixture thereof; or a mixture of two or more thereof.

18. A method for treating a cancer in a subject, comprising administering a therapeutically effective amount of the pharmaceutical formulation according to claim 1 to the subject.

19. A method for preparing a stable pharmaceutical formulation, comprising:
preparing a mixed solution by adding a stabilizer to a solvent; and adding a pembrolizumab or an antigen binding fragment thereof to the mixed solution, or
comprising: preparing a solution of a pembrolizumab or an antigen binding fragment thereof by adding the pembrolizumab or the antigen binding fragment thereof to a solvent; and adding a stabilizer to the solution,
wherein the method is performed without adding a buffer.


SEQUENCE LISTING

<110>  SAMSUNG BIOEPIS CO., LTD.

<120>  Stable anti-PD1 antibody liquid pharmaceutical formulations

<130>  PX063088

<150>  KR 10-2019-0167147
<151>  2019-12-13

<160>  8

<170>  PatentIn version 3.2

<210>  1
<211>  5
<212>  PRT
<213>  Artificial

<220>
<223>  heavy chain variable CDR1

<400>  1

Asn Tyr Tyr Met Tyr
1               5

<210>  2
<211>  17
<212>  PRT
<213>  Artificial

<220>
<223>  heavy chain variable CDR2

<400>  2

Gly Ile Asn Pro Ser Asn Gly Gly Thr Asn Phe Asn Glu Lys Phe Lys
1               5                   10                  15

Asn

<210>  3
<211>  11
<212>  PRT
<213>  Artificial

<220>
<223>  heavy chain variable CDR3

<400>  3

Arg Asp Tyr Arg Phe Asp Met Gly Phe Asp Tyr
1               5                   10

<210>  4
<211>  15

<212>  PRT
<213>  Artificial

<220>
<223>  light chain variable CDR1

<400>  4

Arg Ala Ser Lys Gly Val Ser Thr Ser Gly Tyr Ser Tyr Leu His
1               5                   10                  15


<210>  5
<211>  7
<212>  PRT
<213>  Artificial

<220>
<223>  light chain variable CDR2

<400>  5

Leu Ala Ser Tyr Leu Glu Ser
1               5


<210>  6
<211>  9
<212>  PRT
<213>  Artificial

<220>
<223>  light chain variable CDR3

<400>  6

Gln His Ser Arg Asp Leu Pro Leu Thr
1               5


<210>  7
<211>  446
<212>  PRT
<213>  Artificial

<220>
<223>  heavy chain variable region

<400>  7

Val Gln Leu Val Gln Ser Gly Val Glu Val Lys Lys Pro Gly Ala Ser
1               5                   10                  15


Val Lys Val Ser Cys Lys Ala Ser Gly Tyr Thr Phe Thr Asn Tyr Tyr
            20                  25                  30


Met Tyr Trp Val Arg Gln Ala Pro Gly Gln Gly Leu Glu Trp Met Gly
        35                  40                  45


Gly Ile Asn Pro Ser Asn Gly Gly Thr Asn Phe Asn Glu Lys Phe Lys

50                  55                  60

Asn Arg Val Thr Leu Thr Thr Asp Ser Ser Thr Thr Thr Ala Tyr Met
65                  70                  75                  80

Glu Leu Lys Ser Leu Gln Phe Asp Asp Thr Ala Val Tyr Tyr Cys Ala
                85                  90                  95

Arg Arg Asp Tyr Arg Phe Asp Met Gly Phe Asp Tyr Trp Gly Gln Gly
            100                 105                 110

Thr Thr Val Thr Val Ser Ser Ala Ser Thr Lys Gly Pro Ser Val Phe
        115                 120                 125

Pro Leu Ala Pro Cys Ser Arg Ser Thr Ser Glu Ser Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Lys Thr Tyr Thr Cys Asn Val Asp His Lys Pro
        195                 200                 205

Ser Asn Thr Lys Val Asp Lys Arg Val Glu Ser Lys Tyr Gly Pro Pro
    210                 215                 220

Cys Pro Pro Cys Pro Ala Pro Glu Phe Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser Gln Glu Asp Pro Glu Val
            260                 265                 270

Gln Phe Asn Trp Tyr Val Asp Gly Val Glu Val His Asn Ala Lys Thr
        275                 280                 285

Lys Pro Arg Glu Glu Gln Phe Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Gly Leu Pro Ser Ser Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Gln Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Arg Leu Thr Val Asp Lys Ser Arg Trp
                405                 410                 415

Gln Glu Gly Asn Val Phe Ser Cys Ser Val Met His Glu Ala Leu His
            420                 425                 430

Asn His Tyr Thr Gln Lys Ser Leu Ser Leu Ser Leu Gly Lys
        435                 440                 445

<210>  8
<211>  218
<212>  PRT
<213>  Artificial

<220>
<223>  light chain variable region

<400>  8

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Lys Gly Val Ser Thr Ser
            20                  25                  30

Gly Tyr Ser Tyr Leu His Trp Tyr Gln Gln Lys Pro Gly Gln Ala Pro
        35                  40                  45

Arg Leu Leu Ile Tyr Leu Ala Ser Tyr Leu Glu Ser Gly Val Pro Ala
    50                  55                  60

Arg Phe Ser Gly Ser Gly Ser Gly Thr Asp Phe Thr Leu Thr Ile Ser

65                  70                  75                  80

Ser Leu Glu Pro Glu Asp Phe Ala Val Tyr Tyr Cys Gln His Ser Arg
                85                  90                  95

Asp Leu Pro Leu Thr Phe Gly Gly Gly Thr Lys Val Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215